# EUROPEAN PATENT SPECIFICATION

(11) **EP 1 944 026 B1**
(45) Date of publication and mention of the grant of the patent: **26.06.2013**
(21) Application number: 08152489.4
(22) Date of filing: 15.05.2003
(51) Int. Cl.: A61K 31/135, A61K 31/381, A61P 35/00

(54) **Use of EDG receptor binding agents in cancer**
Verwendung von EDG-Rezeptorbindemitteln bei Krebs
Utilisation d'agents de liaison de récepteur EDG dans un cancer

(30) Priority: 16.05.2002 GB 0211261; 20.06.2002 US 390411 P; 24.07.2002 GB 0217150; 24.02.2003 US 449739 P
(43) Date of publication of application: 16.07.2008
(62) Divisional of application: 03730049.8
(73) Proprietor: Novartis AG, 4056 Basel (CH); Novartis Pharma GmbH, 1230 Wien (AT)
(72) Inventor: Baumruker, Thomas, A 2340, Moedling (AT); Brinkmann, Volker, 79102, Freiburg (DE); La Montagne, Kenneth Richard, Morristown, NJ 07960 (US); Lassota, Peter T, Succasunna, NJ 07876 (US); Mechtcheriakova, Diana, A 1130, Wien (AT); Wood, Jeanette Marjorie, CH 4105, Biel-Benken (CH)
(74) Representative: Rouquayrol, Céline Hélène

(56) References cited:
- EP-A1- 1 431 275
- EP-A1- 1 431 284
- US-A- 5 719 176

## Description

The present invention relates to a use for a sphingosine-1-phosphate (S1P) receptor agonist, particularly in the treatment of cancer.
S1P receptor agonists are accelerating lymphocyte homing (LH) agents which elicit a lymphopenia resulting from a re-distribution, preferably reversible, of lymphocytes from circulation to secondary lymphatic tissue, without evoking a generalized immunosuppression. Naïve cells are sequestered; CD4 and CD8 T-cells and B-cells from the blood are stimulated to migrate into lymph nodes (LN) and Peyer's patches (PP), and thus for example infiltration of cells into transplanted organs is inhibited.
S1P receptor agonists are typically sphingosine analog ues, such as 2-substituted 2-aminopropane-1,3-diol or 2-amino-propanol derivatives, e.g. a compound comprising a group of formula X 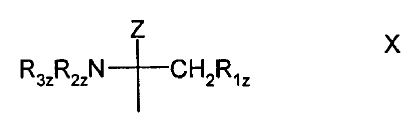 wherein
Z is H; C₁₋₆alkyl; C₂₋₆alkenyl; C₂₋₆alkynyl; phenyl; phenyl substituted by OH; C₁₋₆alkyl substituted by 1 to 3 substituents selected from the group consisting of halogen, C₃₋₈cycloalkyl, phenyl and phenyl substituted by OH; or CH₂-R_{4z} wherein R_{4z} is OH, acyloxy or a residue of formula (a) 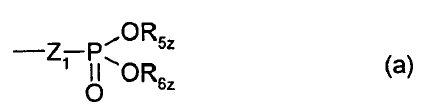 wherein Z₁ is a direct bond or O, preferably O; each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z}, independently, is H, C₁₋₄alkyl or acyl.
Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

S1P receptor agonists are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gβγ-GTP, and/or increased phosphorylation of the agonist-occupied receptor and activation of downstream signaling pathways/kinases. The binding affinity of S1P receptor agonists may be measured as described at paragraph I. below.

### Examples of appropriate S1P receptor agonists are, for example the following (only the compounds of formula IX being part of the invention):

- Compounds as disclosed in EP627406A1, e.g.a compound of formula I 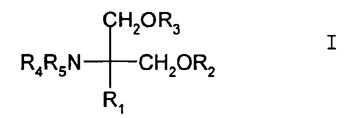
wherein R₁ is a straight- or branched (C₁₂₋₂₂)carbon chain
- which may have in the chain a bond or a hetero atom selected from a double bond, a triple bond, O, S, NR₆, wherein R₆ is H, alkyl, aralkyl, acyl or alkoxycarbonyl, and carbonyl, and/or
- which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
   R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenylalkoxy, halophenylalkoxy, phenylalkoxyalkyl, phenoxyalkoxy or phenoxyalkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
   and wherein
   the alkyl moiety may have
- in the carbon chain, a bond or a heteroatom selected from a double bond, a triple bond, O, S, sulfinyl, sulfonyl, or NR₆, wherein R₆ is as defined above, and
- as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
   each of R₂, R₃, R₄ and R₅, independently, is H, C₁₋₄ alkyl or acyl
   or a pharmaceutically acceptable salt thereof;
- Compounds as disclosed in EP 1002792A1, e.g. a compound of formula II 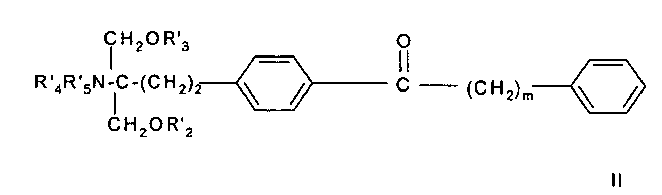
wherein m is 1 to 9 and each of R'₂, R'₃, R'₄ and R'₅, independently, is H, alkyl or acyl,
or a pharmaceutically acceptable salt thereof;
- Compounds as disclosed in EP0778263 A1, e.g. a compound of formula III 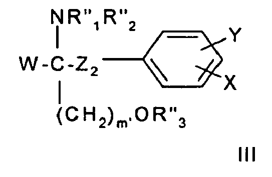
wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆ alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
each of p and q, independently, is an integer of 1 to 20, with the proviso of 6≤p+q≤23, m' is 1, 2 or 3, n is 2 or 3,
each of R"₁, R"₂, R"₃ and R"₄, independently, is H, C₁₋₄alkyl or acyl,
or a pharmaceutically acceptable salt thereof,
- Compounds as disclosed in WO02/18395, e.g. a compound of formula IVa or IVb 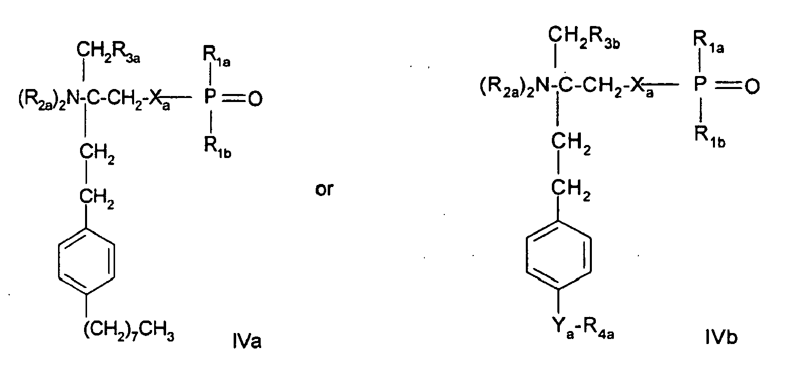
wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
or a pharmaceutically acceptable salt or hydrate thereof;
- Compounds as disclosed in WO 02/076995, e.g. a compound of formula V 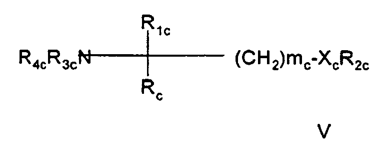
wherein
- m_{c}: is 1, 2 or 3;
- X_{c}: is O or a direct bond;
- R_{1c}: is H; C₁₋₆ alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
- R_{2c}: is 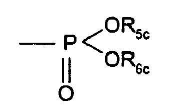 wherein R_{5c} is H or C₁₋₄alkyl optionally substituted by 1, 2 or 3 halogen atoms, and R_{6c} is H or C₁₋₄alkyl optionally substituted by halogen;
each of R_{3c} and R_{4c}, independently, is H, C₁₋₄alkyl optionally substituted by halogen, or acyl, and
- R_{c}: is C₁₃₋₂₀alkyl which may optionally have in the chain an oxygen atom and which may optionally be substituted by nitro, halogen, amino, hydroxy or carboxy; or a residue of formula (a) 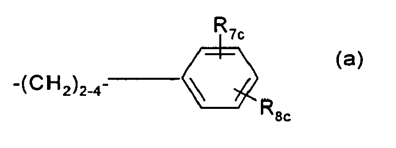 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,
- R_{c}: being also a residue of formula (a) wherein R_{8c} is C₁₋₁₄alkoxy when R_{1c} is C₁₋₄alkyl, C₂₋₆alkenyl or C₂₋₆alkynyl,
or a compound of formula VI 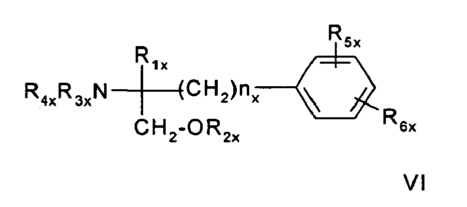 wherein
- nₓ: is 2, 3 or 4
- R₁ₓ: is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
- R₂ₓ: is H, C₁₋₄ alkyl or acyl
each of R₃ₓ and R₄ₓ, independently is H, C₁₋₄alkyl optionally substituted by halogen or acyl,
- R₅ₓ: is H, C₁₋₄alkyl or C₁₋₄alkoxy, and
- R₆ₓ: is C₁₋₂₀alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
- R₆ₓ: being also C₄₋₁₄alkoxy when R₁ₓ is C₂₋₄alkyl substituted by OH, or pentyloxy or hexyloxy when R₁ₓ is C₁₋₄akyl,
provided that R₆ₓ is other than phenyl-butylenoxy when either R₅ₓ is H or R₁ₓ is methyl,
or a pharmaceutically acceptable salt thereof;
- Compounds as disclosed in WO02/06268A1, e.g. a compound of formula VII 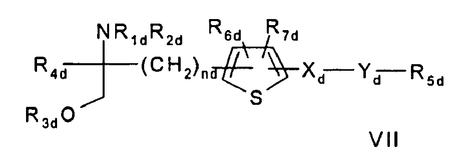
wherein each of R_{1d} and R_{2d}, independently, is H or an amino-protecting group;
R_{3d} is hydrogen or a hydroxy-protecting group;
R_{4d} is lower alkyl;
n_{d} is an integer of 1 to 6;
X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
R_{5d} is hydrogen, cycloalkyl, aryl, heterocycle, cycloalkyl substituted by up to three substituents selected from groups a and b, aryl substituted by up to three substituents selected from groups a and b, or heterocycle substituted by up to three substituents selected from groups a and b; and
each of R_{6d} and R_{7d}, independently, is H or a substituent selected from group a;
<group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano or nitro;
<group b > is cycloalkyl, aryl, heterocycle, each being optionally substituted by up to three substituents selected from group a;
with the proviso that when R_{5d} is hydrogen, Y_{d} is a either a single bond or linear C₁₋₁₀ alkylene, or a pharmacologically acceptable salt or ester thereof.
- Compounds as disclosed in JP-14316985 (JP2002316985), e.g. a compound of formula VIII: 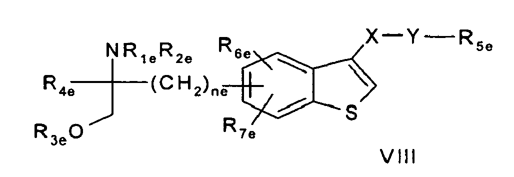
wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt or ester thereof.
- Compounds as disclosed in WO 03/29184 and WO 03/29205, e.g. compounds of formula IX, which are part of the present invention: 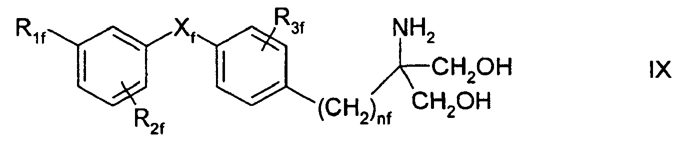
wherein X_{f} is O or S, and R_{1f}, R_{2f}, R_{3f} and n_{f} are as disclosed in WO 03/29184 and O3/29205, e.g. 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol.

Acyl may be a residue Ry-CO- wherein R_{y} is C₁₋₆alkyl, C₃₋₆cycloalkyl, phenyl or phenyl-C₁₄alkyl. Unless otherwise stated, alkyl, alkoxy, alkenyl or alkynyl may be straight or branched.

When in the compounds of formula I the carbon chain as R₁ is substituted, it can be substituted by halogen, nitro, amino, hydroxy or carboxy. When the carbon chain is interrupted by an optionally substituted phenylene, the carbon chain can be unsubstituted. When the phenylene moiety is substituted, it can be substituted by halogen, nitro, amino, methoxy, hydroxy or carboxy.

Compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. R₁ can be phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Each of R₂ to R₅ can be H.

A compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), e.g. the hydrochloride, as shown:

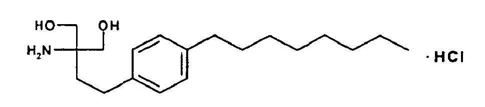

A compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A compound of formula IVa is the FTY720-phosphate (R₂ₐ is H, R₃ₐ is OH, Xₐ is O, R₁ₐ and R_{1b} are OH). A compound of formula IVb is the Compound C-phosphate (R₂ₐ is H, R_{3b} is OH, Xₐ is O, R₁ₐ and R_{1b} are OH, Yₐ is O and R₄ₐ is heptyl). A compound of formula V is Compound B-phosphate.

A compound of formula V is phosphoric acid mono-[(R)-2-amino-2-methyl-4-(4-pentyloxy-phenyl)-butyl]ester.

A compound of formula VIII is (2R)-2-amino-4-[3-(4-cyclohexyloxybutyl)benzo[b]thien-6-yl]-2-methylbutan-1-ol.

When the compounds of formula IX have one or more asymmetric centers in the molecule, the present invention is to be understood as embracing the various optical isomers, as well as racemates, diastereoisomers and mixtures thereof are embraced. Compounds disclosed of formula III or IVb, when the carbon atom bearing the amino group is asymmetric, can have the R-configuration at this carbon atom.

Examples of pharmaceutically acceptable salts of the compounds of the formulae I to IX include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the methods of the present invention encompass hydrate and solvate forms.

The S1P receptor agonists have, on the basis of observed activity, e.g. homing of lymphocytes, e.g. as described in EP627406A1 or USP 6,004,565, been found to be useful e.g. as immunosuppressant, e.g. in the treatment of acute allograft rejection. It has now been found that S1P receptor agonists have interesting properties which make them useful for cancer chemotherapy, particularly of solid tumors, especially of advanced solid tumors. There is still the need to expand the armamentarium of cancer treatment of solid tumors, especially in cases where treatment with anticancer compounds is not associated with disease regression or stabilization.

In the present invention, there is provided:
A use for inhibiting or controlling deregulated angiogenesis, e.g. sphingosine-1-phosphate (S1P) mediated angiogenesis, in a subject in need thereof, comprising administering to said subject a therapeutically effective amount of a S1P receptor agonist comprising a group of formula IX, or a pharmaceutically acceptable salt thereof. and
A use for preventing or treating diseases mediated by a neo-angiogenesis process or associated with deregulated angiogenesis in a subject in need thereof, according to the claims comprising administering to said subject a therapeutically effective amount of a S1P receptor agonist comprising a group of formula IX, or a pharmaceutically acceptable salt thereof.

By "solid tumors" are meant tumors and/or metastasis (whereever located) other than lymphatic cancer, e.g. brain and other central nervous system tumors (eg. tumors of the meninges, brain, spinal cord, cranial nerves and other parts of central nervous system, e.g. glioblastomas or medulla blastomas); head and/or neck cancer; breast tumors; circulatory system tumors (e.g. heart, mediastinum and pleura, and other intrathoracic organs, vascular tumors and tumor-associated vascular tissue); excretory system tumors (e.g. kidney, renal pelvis, ureter, bladder, other and unspecified urinary organs); gastrointestinal tract tumors (e.g. oesophag us, stomach, small intestine, colon, colorectal, rectosigmoid junction, rectum, anus and anal canal), tumors involving the liver and intrahepatic bile ducts, gall bladder, other and unspecified parts of biliary tract, pancreas, other and digestive organs); oral cavity (lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx); reproductive system tumors (e.g. vulva, vagina, Cervix uteri, Corpus uteri, uterus, ovary, and other sites associated with female genital organs, placenta, penis, prostate, testis, and other sites associated with male genital organs); respiratory tract tumors (e.g. nasal cavity and middle ear, accessory sinuses, larynx, trachea, bronchus and lung, e.g. small cell lung cancer or non-small cell lung cancer); skeletal system tumors (e.g. bone and articular cartilage of limbs, bone articular cartilage and other sites); skin tumors (e.g. malignant melanoma of the skin, non-melanoma skin cancer, basal cell carcinoma of skin, squamous cell carcinoma of skin, mesothelioma, Kaposi's sarcoma); and tumors involving other tissues incluing peripheral nerves and autonomic nervous system, connective and soft tissue, retroperitoneum and peritoneum, eye and adnexa, thyroid, adrenal gland and other endocrine glands and related structures, secondary and unspecified malignant neoplasm of lymph nodes, secondary malignant neoplasm of respiratory and digestive systems and secondary malignant neoplasm of other sites.

Where hereinbefore and subsequently a tumor, a tumor disease, a carcinoma or a cancer is mentioned, also metastasis in the original organ or tissue and/or in any other location are implied alternatively or in addition, whatever the location of the tumor and/or metastasis is.

In a series of further specific or alternative embodiments, the present invention also provides
A use for enhancing the activity of a chemotherapeutic agent or for overcoming resistance to a chemotherapeutic agent in a subject in need thereof, comprising administering to said subject a therapeutically effective amount of a S1P receptor agonist , e.g. a S1P receptor agonist comprising a group of formula IX, or a pharmaceutically acceptable salt thereof, either concomitantly or sequentially with said chemotherapeutic agent.
A use according to the previous point wherein the chemotherapeutic agent is an inhibitor of signal transduction pathways directed either against host cells or processes involved in tumor formation and/or metastases formation or utilised by tumour cells for proliferation, survival, differentiation or development of drug resistance.
1.10 A use as indicated above, wherein the S1 P receptor agonist is administered intermittently.

By "lymphatic cancer" are meant e.g. tumors of blood and lymphatic system (e.g. Hodgkin's disease, Non-Hodgkin's lymphoma, Burkitt's lymphoma, AIDS-related lymphomas, malignant immunoproliferative diseases, multiple myeloma and malignant plasma cell neoplasms, lymphoid leukem ia, acute or chronic myeloid leukemia, acute or chronic lymphocytic leukemia, monocytic leukemia, other leukemias of specified cell type, leukemia of unspecified cell type, other and unspecified malignant neoplasms of lymphoid, haematopoietic and related tissues, for example diffuse large cell lymphoma, T-cell lymphoma or cutaneous T-cell lymphoma). Myeloid cancer includes e.g. acute or chronic myeloid leukaemia.

By the term "chemotherapeutic agent" is meant any chemotherapeutic agent other than the S1P receptor agonist according to this list:
i. an aromatase inhibitor,
ii. an antiestrogen, an anti-androgen (especially in the case of prostate cancer) or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin 1 receptor or an angiotensin II antagonist,
vii. a cyclooxygenase inhibitor, a bisphosphonate, a histone deacetylase inhibitor, a heparanase inhibitor (prevents heparan sulphate degradation), e.g. Pl-88, a biological response modifier, preferably a lymphokine or interferons, e.g. interferon γ, an ubiquitination inhibitor, or an inhibitor which blocks anti-apoptotic pathways,
viii. an inhibitor of Ras oncogenic isoforms, e.g. H-Ras, K-Ras or N-Ras, or a farnesyl transferase inhibitor, e.g. L-744,832 or DK8G557,
ix. a telomerase inhibitor, e.g. telomestatin,
x. a protease inhibitor, a matrix metalloproteinase inhibitor, a methionine aminopeptidase inhibitor, e.g. bengamide or a derivative thereof, or a proteosome inhibitor, e.g. PS-341, and/or
xi) a mTOR inhibitor.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN™. Formestane can be administered, e.g., in the form as it is marketed, e.g. under the trademark LENTARON™. Fadrozole can be administered, e.g., in the form as it is marketed, e.g. under the trademark AFEMA™. Anastrozole can be administered, e.g., in the form as it is marketed, e.g. under the trademark ARIMIDEX™. Letrozole can be administered, e.g., in the form as it is marketed, e.g. under the trademark FEMARA™ or FEMAR™ Aminoglutethimide can be administered, e.g., in the form as it is marketed, e.g. under the trademark ORIMETEN™. A combination of the invention comprising a chemotherapeutic agent which is an aromatase inhibitor is particularly useful for the treatment of hormone receptor positive tumors, e.g. breast tumors.

The term "antiestrogen" as used herein relates to a compound which antagonizes the effect of estrogens at the estrogen receptor level. The term includes tamoxifen, fulvestrant, raloxifene and raloxifene hydrochloride. Tamoxifen can be administered, e.g., in the form as it is marketed, e.g. under the trademark NOLVADEX™. Raloxifene hydrochloride can be administered, e.g., in the form as it is marketed, e.g. under the trademark EVISTA™. Fulvestrant can be formulated as disclosed in US 4,659,516 or it can be administered, e.g., in the form as it is marketed, e.g. under the trademark FASLODEX™. A combination of the invention comprising a chemotherapeutic agent which is an antiestrogen is particularly useful for the treatment of estrogen receptor positive tumors, e.g. breast tumors.

The term "anti-androgen" as used herein relates to any substance which is capable of inhibiting the biological effects of androgenic hormones and includes bicalutamide (CASODEX™), which can be formulated, e.g. as disclosed in US 4,636,505.

The term "gonadorelin agonist" as used herein includes abarelix, goserelin and goserelin acetate. Goserelin is disclosed in US 4,100,274 and can be administered, e.g., in the form as it is marketed, e.g. under the trademark ZOLADEX™. Abarelix can be formulated, eg. as disclosed in US 5,843,901.

The term "topoisomerase I inhibitor" as used herein includes, topotecan, irinotecan, 9-nitrocamptothecin and the macromolecular camptothecin conjugate PNU-166148 (compound A1 in WO99/17804). Irinotecan can be administered, e.g. in the form as it is marketed, e.g. under the trademark CAMPTOSAR™. Topotecan can be administered, e.g., in the form as it is marketed, e.g. under the trademark HYCAMTIN™.

The term "topoisomerase II inhibitor" as used herein includes, the anthracyclines such as doxorubicin (including liposomal formulation, e.g. CAELYX™), daunorubicin, epirubicin, idarubicin and nemorubicin, the anthraquinones mitoxantrone and losoxantrone, and the podophillotoxines etoposide and teniposide. Etoposide can be administered, e.g. in the form as it is marketed, e.g. under the trademark ETOPOPHOS™. Teniposide can be administered, e.g. in the form as it is marketed, e.g. under the trademark VM 26-BRISTOL™ Doxorubicin can be administered, e.g. in the form as it is marketed, e.g. under the trademark ADRIBLASTIN™. Epirubicin can be administered, e.g. in the form as it is marketed, e.g. under the trademark FARMORUBICIN™. ldarubicin can be administered, e.g. in the form as it is marketed, e.g. under the trademark ZAVEDOS™. Mitoxantrone can be administered, e.g. in the form as it is marketed, e.g. under the trademark NOVANTRON™.

The term "microtubule active agent" relates to microtubule stabilizing and microtubule destabilizing agents including, taxanes, e.g. paclitaxel and docetaxel, vinca alkaloids, e.g., vinblastine, especially vinblastine sulfate, vincristine especially vincristine sulfate, and vinorelbine, discodermolides and epothilones and derivatives thereof, e.g. epothilone B or a derivative thereof. Paclitaxel may be administered e.g. in the form as it is marketed, e.g. TAXOL™. Docetaxel can be administered, e.g., in the form as it is marketed, e.g. under the trademark TAXOTERE™. Vinblastine sulfate can be administered, e.g., in the form as it is marketed, e.g. under the trademark VINBLASTIN R.P.™. Vincristine sulfate can be administered, e.g., in the form as it is marketed, e.g. under the trademark FARMISTIN™. Discodermolide can be obtained, e.g., as disclosed in US 5,010,099.

The term "alkylating agent" as used herein includes, but is not limited to busulfan, chlorambucil, cyclophosphamide, ifosfamide, melphalan or nitrosourea (BCNU or Gliadel™). Cyclophosphamide can be administered, e.g., in the form as it is marketed, e.g. under the trademark CYCLOSTIN™. Ifosfamide can be administered, e.g., in the form as it is marketed, e.g. under the trademark HOLOXAN™.

The term "antineoplastic antimetabolite" includes, but is not limited to 5-fluorouracil, capecitabine, gemcitabine, cytarabine, fludarabine, thioguanine, methotrexate and edatrexate. Capecitabine can be administered, e.g., in the form as it is marketed, e.g. under the trademark XELODA™. Gemcitabine can be administered, e.g., in the form as it is marketed, e.g. under the trademark GEMZAR™.

The term "platin compound" as used herein includes, but is not limited to carboplatin, cisplatin and oxaliplatin. Carboplatin can be administered, e.g., in the form as it is marketed, e.g. under the trademark CARBOPLAT™. Oxaliplatin can be administered, e.g., in the form as it is marketed, e.g. under the trademark ELOXATIN™.

The term "compounds targeting/decreasing a protein or lipid kinase activity or further anti-angiogenic compounds" as used herein includes, but is not limited to protein tyrosine kinase and/or serine and/or threonine kinase inhibitors or lipid kinase inhibitors, e.g. compounds targeting, decreasing or inhibiting the activity of the epidermal growth factor family of receptor tyrosine kinases (EGFR, ErbB2, ErbB3, ErbB4 as homo- or heterodimers), the vascular endothelial growth factor family of receptor tyrosine kinases (VEGFR), the platelet-derived growth factor-receptors (PDGFR), the fibroblast growth factor-receptors (FGFR), the insulin-like growth factor receptor 1 (IGF-1R), the Trk receptor tyrosine kinase family, the Axl receptor tyrosine kinase family, the Ret receptor tyrosine kinase, the Kit/SCFR receptor tyrosine kinase, members of the c-Abl family and their gene-fusion products (e.g. BCR-Abl), members of the protein kinase C (PKC) and Raf family of serine/threonine kinases, members of the MEK, SRC, JAK, FAK, PDK or PI(3) kinase family, or of the PI(3)-kinase-related kinase family, and/or members of the cyclin-dependent kinase family (CDK) and anti-angiogenic compounds having another mechanism for their activity, e.g. unrelated to protein or lipid kinase inhibition.

Compounds which target, decrease or inhibit the activity of VEGFR are especially compounds, proteins or antibodies which inhibit the VEGF receptor tyrosine kinase, inhibit a VEGF receptor or bind to VEGF, and are in particular those compounds, proteins or monoclonal antibodies generically and specifically disclosed in WO 98/35958, e.g. 1-(4-chloroanilino)-4-(4-pyridylmethyl)phthalazine or a pharmaceutically acceptable salt thereof, e.g. the succinate, in WO 00/27820, e.g. a N-aryl(thio) anthranilic acid amide derivative e.g. 2-[(4-pyridyl)methyl]amino-N-[3-methoxy-5-(trifluoromethyl)phenyl]benzamide or 2-[(1-oxido-4-pyridyl)methyl]amino-N-[3-trifluoromethylphenyl]benzamide, or in WO 00/09495, WO 00/59509, WO 98/11223, WO 00/27819 and EP 0 769 947; those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58, 1998, 3209-3214, and by J. Mordenti et al in Toxicologic Pathology, Vol. 27, no. 1, pp 14-21, 1999; in WO 00/37502 and WO 94/10202; Angiostatin^{™}, described by M. S. O'Reilly et al, Cell 79, 1994, 315-328; Endostatin^{™}, described by M. S. O'Reilly et al, Cell 88, 1997, 277-285; anthranilic acid amides; ZD4190; ZD6474; SU5416; SU6668; or anti-VEGF antibodies or anti-VEGF receptor antibodies,e.g. RhuMab.

By antibody is meant intact monoclonal antibodies, polyclonal antibodies, multispecific antibodies formed from at least 2 intact antibodies, and antibodies fragments so long as they exhibit the desired biological activity.

Compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, or which have a dual inhibiting effect on the ErbB and VEGF receptor kinase and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180) or PCT/EP02/08780; e.g. trastuzumab (Herpetin^{R}), cetuximab, Iressa, OSI-774, CI-1033, EKB-569, GW-2016, E1.1, E2.4, E2.5, E6.2, E6.4, E2.11, E6.3 or E7.6.3.

Compounds which target, decrease or inhibit the activity of PDGFR are especially compounds which inhibit the PDGF receptor, e.g. a N-phenyl-2-pyrimidine-amine derivative, e.g. imatinib.

Compounds which target, decrease or inhibit the activity of c-Abl family members and their gene fusion products, e.g. a N-phenyl-2-pyrimidine-amine derivative, e.g. imatinib; PD180970; AG957; or NSC 680410.

Compounds which target, decrease or inhibit the activity of protein kinase C, Raf, MEK, SRC, JAK, FAK and PDK family members, or PI(3) kinase or PI(3) kinase-related family members, and/or members of the cyclin-dependent kinase family (CDK) are especially those staurosporine derivatives disclosed in EP 0 296 110, e.g. midostaurin; examples of further compounds include e.g. UCN-01, safingol, BAY 43-9006, Bryostatin 1, Perifosine; UO126; Ilmofosine; RO 318220 and RO 320432; GO 6976; Isis 3521; or LY333531/LY379196.

Further anti-angiogenic compounds are e.g. thalidomide (THALOMID) and TNP-470.

Compounds which target, decrease or inhibit the activity of a protein or lipid phosphatase are e.g. inhibitors of phosphatase 1, phosphatase 2A, PTEN or CDC25, e.g. okadaic acid or a derivative thereof.

Compounds which induce cell differentiation processes are e.g. retinoic acid, α-, γ- or δ -tocopherol or α-, γ- or δ-tocotrienol.

The term cyclooxygenase inhibitor as used herein includes, but is not limited to, e.g. celecoxib (Celebrex^{R}), rofecoxib (Vioxx^{R}), etoricoxib, valdecoxib or a 5-alkyl-2-arylaminophenylacetic acid, e.g. 5-methyl-2-(2'-chloro-6'-fluoroanilino)phenyl acetic acid.

The term "histone deacetylase inhibitor" as used herein includes, MS-27-275, SAHA, pyroxamide, FR-901228 or valproic acid.

The term "bisphosphonates" as used herein includes, but is not limited to, etridonic, clodronic, tiludronic, pamidronic, alendronic, ibandronic, risedronic and zoledronic acid. "Etridonic acid" can be administered, e.g., in the form as it is marketed, e.g. under the trademark DIDRONEL™. "Clodronic acid" can be administered, e.g., in the form as it is marketed, e.g. under the trademark BONEFOS™. "Tiludronic acid" can be administered, e.g., in the form as it is marketed, e.g. under the trademark SKELID™. "Pamidronic acid" can be administered, e.g. in the form as it is marketed, e.g. under the trademark AREDIA™. "Alendronic acid" can be administered, e.g., in the form as it is marketed, e.g. under the trademark FOSAMAX™. "lbandronic acid" can be administered, e.g., in the form as it is marketed, e.g. under the trademark BONDRANAT™. "Risedronic acid" can be administered, e.g., in the form as it is marketed, e.g. under the trademark ACTONEL™. "Zoledronic acid" can be administered, e.g. in the form as it is marketed, e.g. under the trademark ZOMETA™

The term "matrix metalloproteinase inhibitor" as used herein includes, but is not limited to collagen peptidom imetic and nonpetidom imetic inhibitors, tetracycline derivatives, e.g. hydroxamate peptidomimetic inhibitor batimastat and its orally bioavailable analogue marimastat, prinomastat, BMS-279251, BAY 12-9566, TAA211 or AAJ996.

The term "mTOR inhibitor" as used herein includes, but is not limited to rapamycin (sirolimus) or a derivative thereof. Rapamycin is a known macrolide antibiotic produced by Streptomyces hygroscopicus. Suitable derivatives of rapamycin include e.g. compounds of formula A 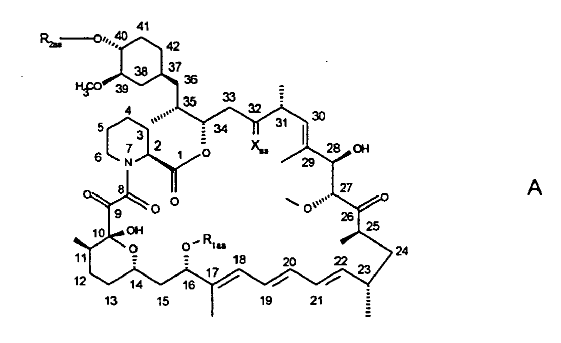 wherein
R₁ₐₐ is CH₃ or C₃₋₆alkynyl,
R₂ₐₐ is H or -CH₂-CH₂-OH, 3-hydroxy-2-(hydroxymethyl)-2-methyl-propanoyl or tetrazolyl, and
Xₐₐ is =O, (H,H) or (H,OH)
provided that R₂ₐₐ is other than H when Xₐₐ is =O and R₁ₐₐ is CH₃.
or a prodrug thereof when R₂ₐₐ is -CH₂-CH₂-OH, e.g. a physiologically
hydrolysable ether thereof.

Compounds of formula A are disclosed e.g. in WO 94/09010, WO 95/16691, WO 96/41807, USP 5,362,718 or WO 99/15530 . They may be prepared as diclosed or by analogy to the procedures described in these references

Preferred rapamycin derivatives are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably,
40-0-(2-hydroxyethyl)-rapamycin. Further examples of rapamycin derivatives include e.g. CC1779 or 40- [3-hydroxy-2-(hydroxymethyl)-2-methylpropanoate]-rapamycin or a pharmaceutically acceptable salt thereof, as disclosed in USP 5,362,718, ABT578 or 40-(tetrazolyl)-rapamycin, particularly 40-epi-(tetrazolyl)-rapamycin, e.g. as disclosed in WO 99/15530, or rapalogs as disclosed e.g. in WO 98/02441 and WO01/14387, e.g. AP23573.

In each case where citations of patent applications or scientific publications are given, the subject-matter relating to the compounds is hereby incorporated into the present application by reference. Comprised are likewise the pharmaceutically acceptable salts thereof, the corresponding racemates, diastereoisomers, enantiomers, tautomers as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e. a pharmaceutical combination within the scope of this invention could include three active ingredients or more. Further both the first agent and the co-agent are not the identical ingredient.
Utility of the S1P agonists, e.g. the S1P agonists comprising a group of formula X according to the claims, in treating solid tumors as hereinabove specified, may be demonstrated in animal test methods as well as in clinic, for example in accordance with the methods hereinafter described.

All the examples are reference examples.

### A. In Vitro

### A.1 Antitumor Activity

A mouse breast cancer cell line originally isolated from mammary carcinomas is used, e.g. JygMC(A). The cell number is adjusted to 5x10⁵ for plating in fresh medium before the procedure. Cells are incubated with fresh medium containing 2.5mM of thymidine without FCS for 12 h and then washed twice with PBS, followed by addition of fresh medium with 10% FCS and additionally incubated for another 12h. Thereafter the cells are incubated with fresh medium containing 2.5mM of thymidine without FCS for 12h. To release the cells from the block, the cells are washed twice with PBS and replated in fresh medium with 10% FCS. After synchronisation, the cells are incubated with or without various concentrations of a compound of formula I for 3, 6, 9, 12, 18 or 24h. The cells are harvested after treatment with 0.2% EDTA, fixed with ice-cold 70% ethanol solution, hydrolyzed with 250µg/ml of RNaseA (type 1-A: Sigma Chem. Co.) at 37°C for 30 mn and stained with propidium iodide at 10mg/ml for 20 mn. After the incubation period, the number of cells is determined both by counting cells in a Coulter counter and by the SRB colorimetric assay. Under these conditions an S1P agonist, e.g. Compound B in hydrochloride salt form, inhibits the proliferation of the tumor cells at concentrations rang ing from 10⁻¹² to 10⁻⁶ M.

### A.2 S1 P-Mediated HUVEC Tube Formation Assay

For the tube formation assay, HUVEC from passage 2-8 are used and are never greater than 70% confluent before harvesting. Cells are prepared for the assay by washing with Herpes Balanced Saline Solution (HBSS from Clonetics) and then trypsinizing with Trypsin/EDTA (0.25 mg/ml, from Clonetics). After approximately 90 % of the cells have lifted off the plate, an equal volume of Trypsin Neutralizing Solution (TNS from Clonetics) is added and the cells are collected into a conical tube containing at least 10 ml of EBM-2 (Clonetics) + 0.1 % BSA (Sigma) media. Cells are centrifuged at 1000 rpm for 5 minutes and the supernatant is removed and replaced with 5 ml of fresh EBM-2 + 0.1 % BSA. Cells are counted using a hemacytometer and the volume of the cell suspension is adjusted to achieve a concentration of 500,000 cells/ml. Conical tubes are prepared with test compounds at 100 nM, and pertussin toxin (PTx) at 10 ng/ml in each, then 1 ml of the cell suspension is added to each tube. Tubes are then incubated for ½ hour at 37 °C, 5 % CO₂. The migration assay is performed using Fluoro-Blok 24-Multiwell Insert Plates coated with fibronectin (8 µm pore size, Falcon #351147) instead of the individual inserts in a 24-well plate. Cells and test compounds are prepared and pre-incubated as described above, then 100 µl is added to each approriate well in the Insert Plate. 300 µl of the EBM-2 + 2 % charcoal-stripped media without S1 P is added to the bottoms of the wells marked for no stimulation (-), and 300 µl of the media containing S1 P (500 nM) is added to the bottoms of the wells marked for stimulation (+). The plate is then incubated for 4 hours at 37 °C, 5 % CO₂.

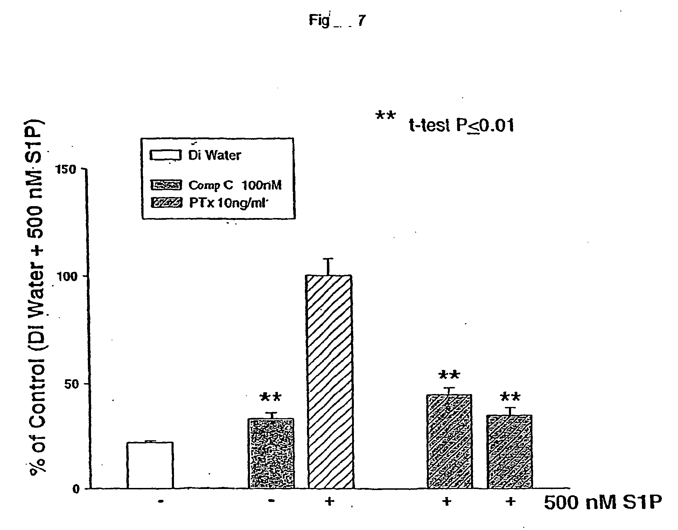

Calcein AM, 50 µg/vial, (Molecular Probes #C3100) is prepared by first adding 20 µl DMSO to the vial. Then 12.5 ml of HBSS (per plate) is warmed to 37 °C and 150 µl is added to the vial. The contents of the vial are then transferred back to the remaining HBSS to make the final concentration 4 µg/ml Calcein AM.
The Fluoro-Blok plate is removed from the incubator and the top insert plate is separated and "flicked" to remove excess media clinging to the inserts. The insert plate is then transferred to a fresh 24-well plate containing 500 µl/well of the 4 µg/ml Calcein AM. The plate is then incubated for 1½ hours at 37 °C, 5 % CO₂.
After incubation, the plate is read on a Cytofluor II at an excitation of 485 nm and emission of 530 nm. The Fluoro-Blok coating in the inserts allows only the cells that have migrated to the bottom to be counted. Data are transferred to Excel for calculations, graphs are created using SigmaPlot, and SigmaStat is used for significance tests (t-test). (Figure 7).
Tube formation is quantitated by counting the number of branching points (two independent cords connecting) in 3 independent fields at 4x magnification. The results are reported as follows:

| Treatment | Branching Points |
|---|---|
| PBS | 8 ± 5 |
| S1P | 42 ± 13 |
| FTY720-Phosphate | 48 ± 15 |
| FTY720-Phosphate + S1P | 14 ± 7 |
| Compound C-Phosphate | 44 ± 16 |
| Compound C-Phosphate + S1P | 18 ± 6 |

These results demonstrate the unique ability of FTY720-Phosphate or Compound C-Phosphate to act as an agonist of angiogenesis on its own, but then surprisingly, as an antagonist of S1P-mediated angiogenesis. Compound C-Phosphate is preferably the racemate or the R-enantiomer. PTx is used as a control to inhibit Giα (EDG-1) mediated activity.

### B. In Vivo

### B.1 Antitumor Activity

Antitumor activity is expressed as T/C% (mean increase in tumor volumes of treated animals divided by the mean increase of tumor volumes of control animals multiplied by 100).

Aliquots of cancer cells (1x10⁷), e.g. human A375 melanoma cells, are transplanted into BALB/c-*nu*/*nu* mice. When the tumors have reached ca. 10x10 mm in size, the animals are assigned random ly to four subgroups and the treatment with a compound of formula I is initiated. Animals are sacrificed after 2 week treatment, at which times tumors and tissues are harvested and prepared for morphological and molecular analysis. The size of the tumors is determined with a caliper. In this assay, an S1 P agonist, e.g. Compound B or C (in the hydrochloride salt form), slows tumor growth when administered at a dose of from 0.5 to 5 mg/kg vs saline control: for example, Compound C-HCl when administered at a dose of 2.5mg/kg 5x/week results in a final T/C value of 30%.

### B.2 Combination with a VEGF-R protein tyrosin kinase inhibitor

Nude mice transplanted with human MDA-MB-435 breast tumors are treated for 2 weeks with a VEGF-R protein tyrosin kinase inhibitor, e.g. 1-(4-chloroanilino)-4-(4-pyridylmethyl)phthalazine succinate, at a dose of 100 mg/kg p.o. 5x/week, a S1P receptor agonist, e.g. Compound C (hydrochloride salt), at a dose of 2.5 mg/kg i.v. 5x/week, or a combination of both. Antitumor is expressed as T/C% as indicated above. A combination of Compound C-HCI with 1-(4-chloroanilino)-4-(4-pyridylmethyl)phthalazine succinate produces a greater antitumor effect (T/C% 27) as compared to either agent alone (Compound C-HCl, T/C 66%; 1-(4-chloroanilino)-4-(4-pyridylmethyl)phthalazine succinate, T/C% 91). Good antitumor responses are also obtained when nude mice are transplanted with human A375 melanoma cells and treated in a similar way with the same combination: the combined treatment results in a T/C% 15 whereas treatment with each agent alone results in a T/C% 35 and 44, respectively.

### B.3 Antiangiogenic Activity

Porous chambers containing (i) sphingosine-1-phosphate (5 µM/chamber) or (ii) human VEGF (1 µg/chamber) in 0.5 ml of 0.8% w/v agar (containing heparin, 20 U/ml) are implanted subcutaneously in the flank of mice. S1 P or VEGF induces the growth of vascularized tissue around the chamber. This response is dose-dependent and can be quantified by measuring the weight and blood content of the tissue. Mice are treated once a day (i) orally with Compound A (0.3, 3, 30 or 50 mg/kg) or (ii) intravenously with the R enantiomer of Compound C (2.5 mg/kg) or (iii) intravenously with the S enantiomer of Compound C (2.5 mg/kg) or (iv) orally or intravenously with vehicle (5% glucose, 10 ml/kg), starting 4-6 hours before implantation of the chambers and continuing for 4 days. The animals are sacrificed for measurement of the vascularized tissues 24 h after the last dose. The weight and blood content of the vascularized tissues around the chamber is determined.

Animals treated with Compound A or with the R or S enantiomer of Compound C show reduced weight and/or blood content of the vascularized tissues compared to animals treated with vehicle alone.

### C. Clinical Trial

### C.1 Investigation of clinical benefit of a S1 P receptor agonist, e.g. a compound of formula I, II or III, e.g. Compound A, B or C

20 patients with progressing, advanced-stage solid tumors, resistant or refractory to standard therapies, to receive said compound at a dosage as determined by a dose escalating study. The general clinical state of the patient is investigated weekly by physical and laboratory examination. Changes in tumor and metastases burden are assessed every 2 months by radiological examination. Initially patients receive treatment for 2 months. Thereafter, they remain on treatment for as long as their disease does not progress and the drug is satisfactorily tolerated.
Main variables for evaluation: Safety (adverse events), standard serum biochemistry and haematology, tumor dimensions by computerised tomographic (CT) scan or magnetic resonance imaging (MRI).

### C.2 Combined Treatment

Suitable clinical studies are, for example, open label non -randomized, dose escalation studies in patients with advanced solid tumors. Such studies prove in particular the synergism of the active ingredients of the combination of the invention. The beneficial effects on proliferative diseases can be determined directly through the results of these studies or by changes in the study design which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and the co-agent (b) is administered with a fixed dose. Alternatively, the agent (a) is administered in a fixed dose and the dose of co-agent (b) is escalated. Each patient receives doses of the agent (a) either daily or intermittent. The efficacy of the treatment can be determined in such studies, e.g., after 12, 18 or 24 weeks by radiologic evaluation of the tumors every 6 weeks.

Alternatively, a placebo-controlled, double blind study can be used in order to prove the benefits of the combination of the invention mentioned herein.

Daily dosages required in practicing the method of the present invention when a S1P receptor agonist alone is used will vary depending upon, for example, the compound used, the host, the mode of administration and the severity of the condition to be treated. A preferred daily dosage range is about from 0.1 to 100 mg as a single dose or in divided doses. Suitable daily dosages for patients are on the order of from e.g. 0.1 to 50 mg p.o. The S1P receptor agonist may be administered by any conventional route, in particular enterally, e.g. orally, e.g. in the form of tablets, capsules, drink solutions, nasally, pulmonary (by inhalation) or parenterally, e.g. in the form of injectable solutions or suspensions. Suitable unit dosage forms for oral administration comprise from ca. 0.1 to 30 mg, usually 0.25 to 30 mg S1 P receptor agonist, together with one or more pharmaceutically acceptable diluents or carriers therefore. In order to inhibit angiogenesis it is important to select a sufficiently high dose of the S1 P receptor agonist, as low concentrations of S1 P receptor agonists promote angiogenesis. A suitable dose for providing an anti-angiogenic effect when a S1 P agonist is administered to a patient may be selected by concentration- and dose-escalating studies as described at A, B, and C above.

The combination of the invention can also be applied in combination with surgical intervention, mild prolonged whole body hyperthermia and/or irradiation therapy.

The administration of a pharmaceutical combination of the invention results in a beneficial effect, e.g. a synergistic therapeutic effect, e.g. with regard to slowing down, arresting or reversing the neoplasm formation, metastases spread or growth or a longer duration of tumor response or inhibition of angiogenesis; it may also result in other beneficial effects, e.g. less side-effects, an improved quality of life or a decreased mortality and morbidity, compared to a monotherapy applying only one of the pharmaceutically active ingredients used in the combination of the invention, in particular in the treatment of a tumor that is refractory to other chemotherapeutics known as anti-cancer agents.

A further benefit is that lower doses of the active ingredients of the combination of the invention can be used, for example, that the dosages need not only often be smaller but are also applied less frequently, or can be used in order to diminish the incidence of side-effects, while controlling the growth of neoplasm formation. This is in accordance with the desires and requirements of the patients to be treated.

According to one embodiment of the invention, a preferred pharmaceutical combination comprises
a) a compound of formula I, II, III, IVa, IVb, V or VI, e.g. Compound A, B or C, and
b) as a co-agent, one or more compounds as indicated in paragraphs (ii), (iii), (iv), (v), (vii) or (xi) above, e.g. carboplatin, cisplatinum, paclitaxel, docetaxel, gemcitabine, doxorubicin, a compound targeting, decreasing or inhibiting the activity of the vascular endothelial growth factor family of receptor tyrosine kinases (VEGFR) or the platelet-derived growth factor-receptors (PDGFR), a bisphosphonate or a mTOR inhibitor.

A further embodiment of the invention relates to the use of S1 P receptor agonist (a) in combination with a chemotherapeutic agent (b) in the treatment of a lymphatic or myeloid cancer, e.g. as disclosed above. The combination may comprise as a further co-agent b) e.g. busulfan, cytarabine, 6-thioguanine, fludarabine, hydroxyurea, procarbazine, bleomycin or methotrexate. Topoisomerase II inhibitors e,g. daunorubicin or, particularly, compounds which target, decrease or inhibit the activity of PDGFR or of c-Abl family members and their gene fusion products, e.g. imatinib, are preferred as co-agent (b), e.g. for use in the treatment of a lymphatic cancer.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

It is one objective of this invention to provide a pharmaceutical composition comprising a quantity, which is jointly therapeutically effective against a proliferative malignant disease comprising a combination of the invention. In this composition, the first agent a) and co-agent (b) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of delay of progression or treatment of a proliferative malignant disease according to the invention may comprise (i) administration of the first agent a) in free or pharmaceutically acceptable salt form and (ii) administration of a co-agent b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

Daily dosages for the first agent or component (a) will, of course, vary depending on a variety of factors, for example the compound chosen, the particular condition to be treated and the desired effect. In general, however, satisfactory results are achieved on administration of a S1 P receptor agonist, e.g. Compound A, B or C, at daily dosage rates of the order of ca. 0.1 to 100 mg as a single dose or in divided doses. The S1 P receptor agonist may be administered by any conventional route, in particular enterally, e.g. orally, e.g. in the form of tablets, capsules, drink solutions or parenterally, e.g. in the form of injectable solutions or suspensions. Suitable unit dosage forms for oral administration comprise from ca. 0.1 to 30 mg component (a), e.g. 0.1 to 25 mg, together with one or more pharmaceutically acceptable diluents or carriers therefor.

Fadrozole may be administered orally to a human in a dosage range varying from about 0.5 to about 10 mg/day, preferably from about 1 to about 2.5 mg/day. Exemestane may be administered orally to a human in a dosage range varying from about 5 to about 200 mg/day, preferably from about 10 to about 25 mg/day, or parenterally from about 50 to 500 mg/day, preferably from about 100 to about 250 mg/day. If the drug shall be administered in a separate pharmaceutical composition, it can be administered in the form disclosed in GB 2,177,700. Formestane may be administered parenterally to a human in a dosage range varying from about 100 to 500 mg/day, preferably from about 250 to about 300 mg/day. Anastrozole may be administered orallly to a human in a dosage range varying from about 0.25 to 20 mg/day, preferably from about 0.5 to about 2.5 mg/day. Aminogluthemide may be administered to a human in a dosage range varying from about 200 to 500 mg/day.

Tamoxifen citrate may be administered to a human in a dosage range varying from about 10 to 40 mg/day.

Vinblastine may be administered to a human in a dosage range varying from about 1.5 to 10 mg/m²day. Vincristine sulfate may be administered parenterally to a human in a dosage range varying from about 0.025 to 0.05 mg/kg body weight * week. Vinorelbine may be administered to a human in a dosage range varying from about 10 to 50 mg/m²day. Etoposide phosphate may be administered to a human in a dosage range varying from about 25 to 115 mg/m²day, e.g. 56.8 or 113.6 mg/m²day.

Teniposide may be administered to a human in a dosage range varying from about 75 to 150 mg about every two weeks. Doxorubicin may be administered to a human in a dosage range varying from about 10 to 100 mg/m²day, e.g. 25 or 50 mg/m²day. Epirubicin may be administered to a human in a dosage range varying from about 10 to 200 mg/m²day. Idarubicin may be administered to a human in a dosage range varying from about 0.5 to 50 mg/m²day. Mitoxantrone may be administered to a human in a dosage range varying from about 2.5 to 25 mg/m²day.

Paclitaxel may be administered to a human in a dosage range varying from about 50 to 300 mg/m²day. Docetaxel may be administered to a human in a dosage range varying from about 25 to 100 mg/m²day.

Cyclophosphamide may be administered to a human in a dosage range varying from about 50 to 1500 mg/m²day. Melphalan may be administered to a human in a dosage range varying from about 0.5 to 10 mg/m²day.

5-Fluorouracil may be administered to a human in a dosage range varying from about 50 to 1000 mg/m²day, e.g. 500 mg/m²day. Capecitabine may be administered to a human in a dosage range varying from about 10 to 1000 mg/m²day. Gemcitabine hydrochloride may be administered to a human in a dosage range varying from about 1000 mg/m²/week. Methotrexate may be administered to a human in a dosage range varying from about 5 to 500 mg/m²day.

Topotecan may be administered to a human in a dosage range varying from about 1 to 5 mg/m²day. Irinotecan may be administered to a human in a dosage range varying from about 50 to 350 mg/m²day.

Carboplatin may be administered to a human in a dosage range varying from about 200 to 400 mg/m² about every four weeks. Cisplatin may be administered to a human in a dosage range varying from about 25 to 75 mg/m² about every three weeks. Oxaliplatin may be administered to a human in a dosage range varying from about 50 to 85 mg/m² every two weeks.

Imatinib may be administered to a human in a dosage in the range of about 2.5 to 850 mg/day, more preferably 5 to 600 mg/day and most preferably 20 to 300 mg/day.

Alendronic acid may be administered to a human in a dosage range varying from about 5 to 10 mg/day. Clodronic acid may be administered to a human e.g. in a dosage range varying from about 750 to 1500 mg/day. Etridonic acid may be administered to a human in a dosage range varying from about 200 to 400 mg/day. lbandronic acid may be administered to a human in a dosage range varying from about 1 to 4 mg every three to four weeks.
Risedronic acid may be administered to a human in a dosage range varying from about 20 to 30 mg/day. Pamidronic acid may be administered to a human in a dosage range varying from about 15 to 90 mg every three to four weeks. Tiludronic acid may be administered to a human in a dosage range varying from about 200 to 400 mg/day.

Trastuzumab may be administered to a human in a dosage range varying from about 1 to 4 mg/m²/week.

Bicalutamide may be administered to a human in a dosage range varying from about 25 to 50 mg/m²day.

1-(4-chloroanilino)-4-(4-pyridylmethyl)phthalazine or salt thereof, e.g. succinate, may be administered to a human in a dosage range of about 50 to 1500, more preferably about 100 to 750, and most preferably 250 to 500, mg/day.

Rapamycin or a derivative thereof, e.g. 40-O-(2-hydroxyethyl)-rapamycin, may be administered in a dosage range varying from about 0.1 to 25 mg.

Formulation Example: soft capsules

| | |
|---|---|
| Compound of formula I, | |
| e.g. Compound A, HCl | 30 mg |
| Polyethylene glycol 300 | 300 mg |
| Polysorbate 80 | 20 mg |
| Total | 350 mg |

The S1 P receptor agonists, e.g. a S1 P receptor agonist comprising a group of formula X, are well tolerated at dosages required for use in accordance with the present invention. For example, the acute LD₅₀ for Compound A is > 10 mg/kg p.o. in rats and monkeys.

In a further aspect, the present invention relates to the use of S1 P agonists as pro-angiogenic drugs. Induction of neo-angiogenesis has lately been recognized as an excellent target in a number of conditions (e.g.myocardial angiogenesis, wound healing or diabetic vascular dysfunction/vasculopathy).

As described above, high concentrations of S1 P receptor agonists (2 µM or greater, e.g. 2-5 µM or around 5 µM) exhibit anti-angiogenic effects, and S1 P receptor agonists can inhibit VEGF-induced ang iogenesis. In contrast, low concentrations (0.1 -1 µM, e.g. 0.1 - 0.5 µM or 0.5 - 1 µM) of S 1 P agonists have an enhancing effect on ang iogenesis and are able to potentiate VEGF-mediated angiogenesis. Thus, S1P agonists may have biphasic effects in angiogenesis.

Accordingly, the present invention further provides:
8. Use of a S1 P agonist, e.g. a S1 P agonist comprising a group of formula X, e.g. Compound A or Compound A-phosphate, in the induction of the neo-angiogenesis process, e.g. as a pro-angionenic agent, e.g. in indications where a promotion of angiogenesis is indicated;
9. A process for the preparation of a medicament for the treatment or prevention of diseases mediated by the inhibition of the neo-angiogenesis process, e.g. mediated by anti-angionenic factors, e.g. in indications where a promotion of angiogenesis is indicated, e.g. in wound healing or in the treatment of myocardial infarction or diabetic vascular dysfunction/vasculopathy, comprising using a S1 P receptor agonist, e.g. a S1 P agonist comprising a group of formula X, e.g. Compound A or Compound A-phosphate, as an active ingredient;
10. A method of treating or preventing diseases mediated by the inhibition of the neo-angiogenesis process, e.g. mediated by anti-angionenic factors, e.g. in indications where a promotion of angiogenesis is indicated, such as e.g. in wound healing or in the treatment of myocardial infarction or diabetic vascular dysfunction/vasculopathy, comprising administering an effective amount of a S1 P receptor agonist, e.g. a S1 P agonist comprising a group of formula X, e.g. Compound A or Compound A-phosphate, to a subject in need of such treatment.

S1P agonists suitable for promoting angiogenesis include those defined above in relation to the treatment of cancer, e.g. S1 P agonists comprising a group of formula X or compounds according to formulae I to IX, or pharmaceutically acceptable salts or esters thereof. Preferably the S1 P agonist is Compound A-phosphate. The S1 P agonist may be used alone, or in combination with one or more further agents which promote angiogenesis, e.g. VEGF.

In order to promote angiogenesis it is important to select a sufficiently low dose of the S1 P receptor agonist, as high concentrations of S1 P receptor agonists inhibit angiogenesis. A suitable dose for providing a pro-angiogenic effect when a S1 P agonist is administered to a patient may be selected by concentration- and dose-escalating studies as described at A, B, and C above.

### Description of the Figures

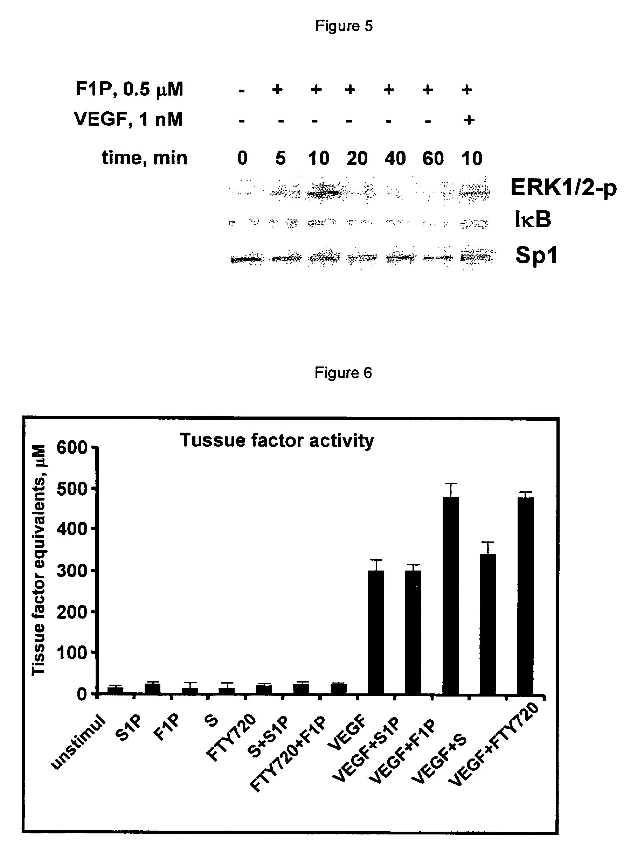

Figure 1
   shows that Compound A-phosphate strongly promotes capillary-like network formation in a bell-shape dose-dependent manner showing maximal activity around 0.5 µM.
Figure 2
   shows that both Compound A-phosphate and Compound A at 0.5 - 1 µM do not attenuate VEGF-mediated remodelling but rather cooperate with polypeptide growth factor.
Figure 3
   Shows that Compound A-phosphate as well as S1P-stimulated tube formation is practically completely inhibited by pertussis toxin (PTX, 50 ng/ml), an inhibitor of heterotrimeric G proteins of α_{i/o}-type. This may be interpreted as a possible involvement of EDG-1 (S1P₁) receptor-mediated signaling events in Compound A-phosphate-stimulated bioresponses.
Figure 4
   Shows, that sphingosine at 1 µM, which itself seems to be less potent than S1 P, attenuates the ability of both S 1 P and Compound A-phosphate to induce capillary-like structures, without having an inhibitory effect on the VEGF-induced tube formation. In this respect, sphingosine behaves different from Compound A. The data indicate that the balance between sphingosine and S1 P seems to be critically important for endothelial cell activation/angiogenesis most likely via the EDG receptor family. Importantly, high concentrations of sphingosine and Compound A (2 - 5 µM) inhibits VEGF-triggered tube formation.
Figure 5
   Shows that the treatment of HUVEC with Compound A-phosphate at 0.5 µM may result in transient activation of ERK1/2 with a peak of phosphorylation/activation at 10 minutes and returning to baseline by 20 minutes
Figure 6
   It was tested whether Compound A, Compound A-phosphate, sphingosine or S1P also do induce tissue factor on HUVEC. The data found demonstrate that none of these compounds alone or in combinations may elevate tissue factor activity as shown in Figure 6. Compound A and Compound A-phosphate may slightly enhance the VEGF- but not TNF-α-induced tissue factor.
Figure 7
   Shows the effect of Compound C in a S1 P-mediated HUVEC tube formation assay.

The following abbreviations are used:

| | |
|---|---|
| BSA: bovine serum albumine | |
| ECGS: endothelial cell growth factor set | ECL: enhanced chemiluminescence |
| S: sphingosine | PBS: phosphate-buffered saline |
| JNK1/2: c-jun-N-terminal kinase1/2 | RT: room temperature |
| TF equivalents: tissue factor equivalents | |
| EGR-1/NFAT: early growth response protein 1/nuclear factor of activated T-cells F1 P: Compound A-phosphate (FTY720-phosphate) | |

Utility of the S1P receptor agonists, e.g. the S1P agonists comprising a group of formula X, in the promotion of angiogenesis may be demonstrated for example in accordance with the methods described hereinafter.

### D. Cell culture and Materials

Human umbilical vein endothelial cells (HUVEC) are cultured at 37°C and 5% CO₂ in medium M199 supplemented with 20% SCS (HyClone, Logan, UT), 1U/ml heparin, 50 µg/ml ECGS, 2 mM glutamine, 100 U/ml penicillin and 0.1 mg/ml streptomycin. Cells are used for experiments up to passage number 5. Short-starved HUVEC are obtained by starving with 1% SCS-containing M199 for 5 h. Recombinant human VEGF₁₆₅ is obtained from PromoCell (Heidelberg, Germany). Phospho-specific ERK1/2, p38 kinase polyclonal antibodies, nonphospho ERK1/2 antibodies and LumiGLO chemiluminescent reagent are from New England BioLabs (Beverly, MA), polyclonal IκB antibodies from Santa Cruz Biotechnology (Santa Cruz, Calif.). Peroxidase-conjugated donkey anti-rabbit immunoglobulin G (IgG) and sheep anti-mouse IgG are purchased from Amersham LIFE SCIENCE (Amersham Place, England). Immobilon-P transfer membranes are products of Millipore (Bedford, MA). S is obtained from Sigma Chemical Co.; S1 P is from Biomol. Compound A-phosphate stock solution is prepared by the following protocol. Compound A-phosphate is dissolved in methanol tracing with concentrated HCl (0.5 mg Compound A-phosphate in 500 µl of methanol plus 2 µl of HCl). Solvent from the resulting solution is evaporated under vacuum and the residue obtained is redissolved (variant 1) in 0.1 % of defatted BSA solution in sterile deionized water (500 µl) or (variant 2) in 0.5 % Triton X-1 00 in deionized water. The resulting stock solutions (2.5 mM) are sonicated and stored at 4°C.

### Clotting Assay

Cells are seeded in 6-well plates at 80-90% confluency and grown overnight. Cells are scraped from the plates and analyzed for tissue factor activity according to the method as described in Clauss, M., J. Biol. Chem. 271,17629-17634 (1996), Mechtcheriakova, D., Blood 93,3811-3823 (1999). Briefly, after induction for 4 hours with VEGF (1.5 nM), TNF-α (100 U/ml), S (0.5-2 µM), S1P (0.5-2 µM), Compound A (0.5-2 µM), and Compound A-phosphate (0.5-2 µM), cells are washed twice and then scraped in 1ml clotting buffer (12 mM sodium acetate, 7 mM diethylbarbitate and 130 mM sodium chloride; pH 7.4). 50 µl of resuspended cells are mixed with 50 µl of citrated plasma, and clotting times are determined after recalcification with 50 µl of 20 mM CaCl₂ solution at 37°C. TF-equivalents are determined by using a standard curve obtained from rabbit brain thromboplastin.

### E. Western Blot Analysis

After various treatments, the cells are washed twice with cold PBS, lysed in 100 µl of Laemmli buffer, scraped and heated for 5 min at 95°C. Total cell lysates are separated by SDS-PAGE and transferred to Immobilon-P membrane. The membrane is blocked for 30 minutes with PBS containing 0.1% Tween-20 and 3% skim milk and incubated for 1 hour at RT with a primary antibody diluted in blocking buffer. The membrane obtained is washed three times for 5 minutes with PBS containing 0.1% Tween-20 and incubated with peroxidase-conjugated secondary antibody for 1 hour at RT. After a washing step, the membrane is incubated for 1 minute with ECL reagent and exposed to film as required. For reprobing with another antibody, the membrane is washed twice in PBS, stripped for 30 min at 55°C with stripping buffer (62.5 mM Tris-HCL, pH 6.8, 2% SDS, 100 mM 2-mercaptoethanol) and washed three times for 5 minutes with PBS at RT. The membrane is stored wet wrapped in SaranWrap at 4°C after each immunodetection.

### In vitro angiogenesis assay on Matrigel

Morphogenesis of endothelial cells into capillary-like structures on Growth Factor Reduced Matrigel Matrix (BD Bioscience) is performed according to the manufacture procedure. Briefly, HUVEC are trypsinized, resuspended in serum-free M199 medium containing soybean trypsin inhibitor (1mg/ml, Sigma). After centrifugation cells are resuspended in serum-free medium at a density 0.5 x 10⁵ cells/ml, and cell suspension is seeded in 96-well cell culture plates (Costar, Corning Incorporated) precoated with 50 µl of Matrigel in the absence or presence of various stimuli: VEGF at 1.5 nM, S1P at 0.1-2 µM, S at 0.5-2 µM, Compound A at 0.5-2 µM, and Compound A-phosphate at 0.1-2 µM. Eight hours later, cells on Matrigel are fixed with 3 % formaldehyde in PBS and kept at 4°C. Results are quantitated from images made with a Nikon Diaphot microscope equipped with a cooled CCD camera (Kappa GmbH, Gleichen, Germany) by direct counting of branching points on two microscopic fields from each well done in duplicates.

### F. Compound A-phosphate-induces morphogenesis of endothelial cells in in vitro tube formation assay on Matrigel and a possible involvment of Gᵢ-mediated signaling pathway(s)

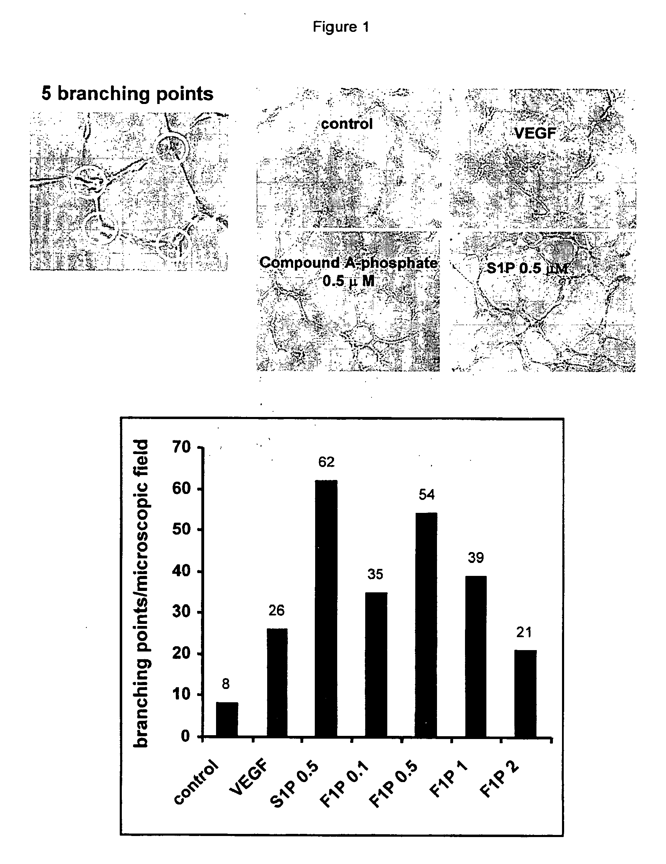

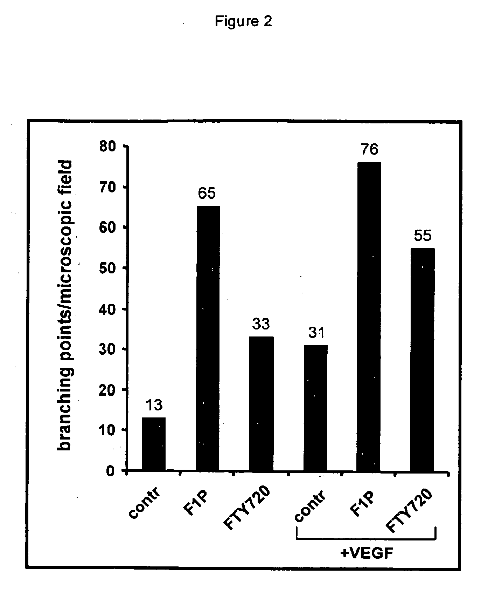

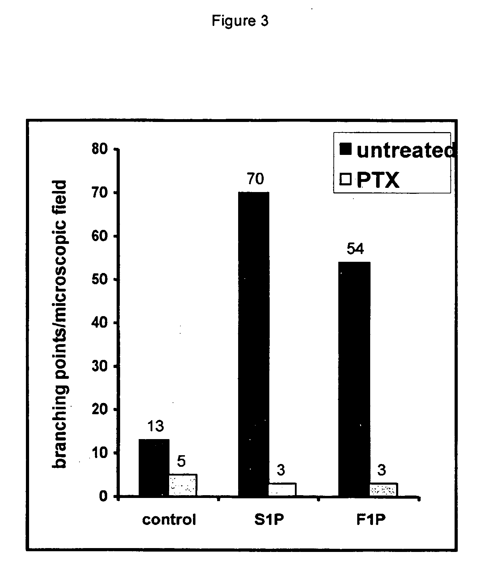

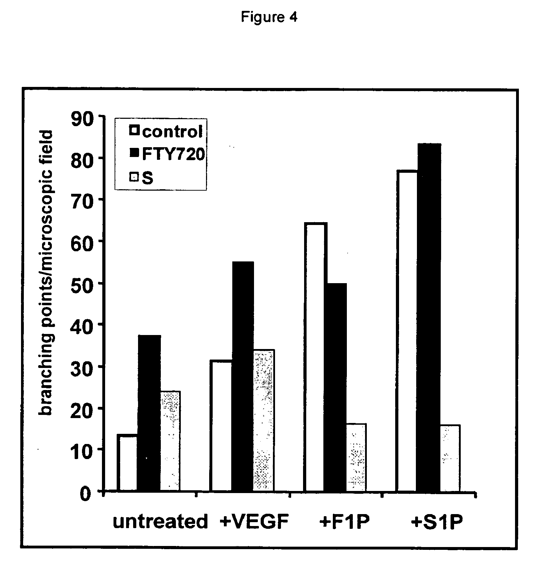

The effect of Compound A and Compound A-phosphate on morphogenic differentiation of endothelial cells is determined using an in vitro angiogenesis assay on Matrigel. Endothelial cell morphogenesis is a complex process that requires cell-extracellular matrix interactions, followed by matrix remodelling, stimulated migration, cell-cell interactions, and perivascular proteolysis. As shown in Figure 1, Compound A-phosphate strongly may promote capillary-like network formation in a bell-shape dose-dependent manner showing maximal activity around 0.5 µM. The number of branching points per microscopic field, which reflects the induction potency of the stimulus, is comparable for Compound A-phosphate and S1P, and may exceed significantly the VEGF-triggered effects. Compound A itself at 0.5 - 1 µM has a weak, in comparison to Compound A-phosphate, but consistent enhancing effect. Both Compound A-phosphate and Compound A at 0.5 - 1 µM does not attenuate VEGF-mediated remodelling but rather cooperates with polypeptide growth factor (see e.g. Figure 2). Furthermore, Compound A-phosphate- as well as S1P-stimulated tube formation is completely inhibited by pertussis toxin (PTX, 50 ng/ml), an inhibitor of heterotrimeric G proteins of α_{i/o}-type. This may be interpreted as a possible involvement of EDG-1 (S1P₁) receptor-mediated signaling events in Compound A-phosphate-stimulated bioresponses (see e.g. Figure 3). S at 1 µM, which itself seems to be less potent than S1 P, attenuates the ability of both S1P and Compound A-phosphate to induce capillary-like structures, without having an inhibitory effect on the VEGF-induced tube formation (see e.g. Figure 4). In this respect, S behaves differently from Compound A. The data indicate that the balance between S and S1 P seems to be critically important for endothelial cell activation/angiogenesis most likely via the EDG receptor family. Importantly, high concentrations of S and Compound A (2 - 5 µM) inhibited VEGF-triggered tube formation. That data suggest biphasic dose-dependent effects of Compound A and Compound A-phosphate on angiogenesis in vitro.

### G. Activation of ERK1/2 MAP kinases by Compound A-phosphate

Signal transduction via MAP kinases plays a key role in a variety of endothelial cell functions. Treatment of HUVEC with Compound A-phosphate at 0.5 µM may result in transient activation of ERK1/2 with a peak of phosphorylation/activation at 10 minutes and returning to baseline by 20 minutes (see e.g. Figure 5). No activation of p38 kinase and JNK1/2 by Compound A-phosphate is detectable in HUVEC. Furthermore, Compound A-phosphate may trigger ERK1/2 activation in a dose-dependent manner, showing stronger activity at 2 µM. This is in contrast to the results from the tube formation assay, where Compound A-phosphate at 2 µM may be less potent than at 0.5 µM. Neither Compound A nor S are able to induce MAP kinase activation in endothelial cells in a kinetics ranging from 5 minute to 60 minute treatment. To estimate the possible role of inflammatory/NFκB-dependent program in Compound A-phosphate-stimulated bioresponses of endothelial cells, the membranes are reprobed with anti-IxB antibodies. IκB levels are not affected by Compound A-phosphate treatment. Moreover the treatment of endothelial cells with Compound A-phosphate may fail to induce E-Selectin expression as a NFκB-dependent secondary responsive gene. Thus, the data strongly indicate that Compound A-phosphate signaling does not involve NFκB activation - the main cascade in the acute inflammatory response in endothelial cells.

### H. Compound A and Compound A-phosphate do not induce tissue factor expression on endothelial cells

An important characteristic feature of both classical inflammatory stimulus TNF-α and the main angiogenic growth factor VEGF on endothelial cells is their potency to upregulate tissue factor. Compound A, Compound A-phosphate, S or S1P are tested whether they also do induce tissue factor on HUVEC. The data found demonstrate that none of these compounds alone or in combinations may elevate tissue factor activity (see e.g. Figure 6). Compound A and Compound A-phosphate may slightly enhance the VEGF- but not TNF-α-induced tissue factor. The data obtained together indicate that Compound A, Compound A-phosphate, S and S1P mechanistically work distinctly to angiogenic VEGF and inflammatory TNF-α.

### I. Binding affinity of S1P receptor agonists to individual human S1P receptors may be determined in following assays:

### Transient transfection of human S1 P receptors into HEK293 cells

EDG receptors and Gᵢ proteins are cloned, and equal amounts of 4 cDNAs for the EDG receptor, Gᵢ-α, Gᵢ-β and Gᵢ-γ are mixed and used to transfect monolayers of HEK293 cells using the calcium phosphate precipitate method (M. Wigler et al., Cell. 1977;11;223 and DS. Im et al., Mol. Pharmacol. 2000;57;753). Briefly, a DNA mixture containing 25 µg of DNA and 0.25 M CaCl is added to HEPES-buffered 2 mM Na₂HPO₄. Subconfluent monolayers of HEK293 cells are poisoned with 25 mM chloroquine, and the DNA precipitate is then applied to the cells. After 4 h, the monolayers are washed with phosphate-buffered saline and refed media (90% 1:1 Dulbecco's modified essential media (DMEM):F-12 + 10% fetal bovine serum). The cells are harvested 48-72 h after addition of the DNA by scraping in HME buffer (in mM: 20 HEPES, 5 MgCl₂, 1 EDTA, pH 7.4) containing 10% sucrose on ice, and disrupted using a Dounce homogenizer. After centrifugation at 800×g, the supernatant is diluted with HME without sucrose and centrifuged at 100,000×g for 1h. The resulting pellet is rehomogenized and centrifuged a second hour at 100,000×g. This crude membrane pellet is resuspended in HME with sucrose, aliquoted, and snap-frozen by immersion in liquid nitrogen. The membranes are stored at 70°C. Protein concentration is determined spectroscopically by Bradford protein assay.

### GTPγS binding assay using S1P receptor/HEK293 membrane preparations

GTPγS binding experiments are performed as described by DS. Im et al., Mol. Pharmacol. 2000; 57:753. Ligand-mediated GTPγS binding to G-proteins is measured in GTP binding buffer (in mM: 50 HEPES, 100 NaCl, 10 MgCl₂, pH 7.5) using 25 µg of a membrane preparation from transiently transfected HEK293 cells. Ligand is added to membranes in the presence of 10 µM GDP and 0.1 nM [³⁵S]GTPγS (1200 Ci/mmol) and incubated at 30°C for 30 min. Bound GTPγS is separated from unbound using the Brandel harvester (Gaithersburg, MD) and counted with a liquid scintillation counter.

## Claims

1. A S1 P receptor agonist which is a compound of formula IX 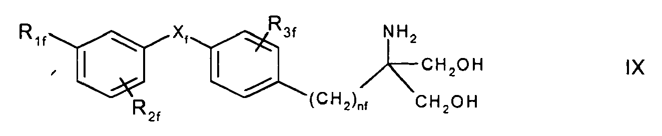 wherein
R_{1f} is halogen, trihalomethyl, hydroxy, lower alkyl having 1 to 7 carbon atoms, phenyl, aralkyl, lower alkoxy having 1 to 4 carbon atoms, trifluoromethyloxy, phenoxy, cyclohexylmethyloxy, substituted or unsubstituted aralkyloxy, pyridylmethyloxy, cinnamyloxy, naphthylmethyloxy, phenoxymethyl, hydroxymethyl, hydroxyethyl, lower alkylthio having 1 to 4 carbon atoms, lower alkylsulfinyl having 1 to 4 carbon atoms, lower alkylsulfonyl having 1 to 4 carbon atoms, benzylthio, acetyl, nitro or cyano;
R_{2f} is hydrogen, halogen, trihalomethyl, lower alkoxy having 1 to 4 carbon atoms, lower alkyl having 1 to 7 carbon atoms, phenethyl, or benzyloxy;
R_{3f} is hydrogen, halogen, trifluoromethyl, lower alkoxy having 1 to 4 carbon atoms, hydroxy, benzyloxy, lower alkyl having 1 to 7 carbon atoms, phenyl or lower alkoxymethyl having 1 to 4 carbon atoms;
X_{f} is O or S; and
n_{f} is an integer from 1 to 4,
or a pharmacologically acceptable salt thereof,
for use in a method for preventing or treating diseases mediated by a neo-angiogenesis process or associated with deregulated angiogenesis, where said diseases are selected from the group consisting of cancer, myocardial angiogenesis, wound healing and diabetic vascular dysfunction or vasculopathy.

2. A S1P receptor agonist for use according to claim 1 for preventing or treating solid tumors.

3. A S1P receptor agonist for use according to claim 1 for inhibiting growth of solid tumors or treating solid tumor invasiveness or symptoms associated with such tumor growth.

4. A S1P receptor agonist of the formula IX as defined in claim 1, or a pharmaceutically acceptable salt thereof, for use in a method for preventing or treating diseases mediated by a neo-angiogenesis process or associated with deregulated angiogenesis, where said diseases are selected from the group consisting of cancer, myocardial angiogenesis, wound healing, diabetic vascular dysfunction or vasculopathy, lymphoproliferative and myeloproliferative disorders, wherein the S1 P receptor agonist is administered concomitantly or in sequence with a chemotherapeutic agent.

5. A S1P receptor agonist for use according to claim 4, wherein the chemotherapeutic agent is selected from
i. an aromatase inhibitor,
ii. an antiestrogen, an anti-androgen or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin 1 receptor or an angiotensin II antagonist,
vii. a cyclooxygenase inhibitor, a bisphosphonate, a histone deacetylase inhibitor, a heparanase inhibitor, a biological response modifier, an ubiquitination inhibitor, or an inhibitor which blocks anti-apoptotic pathways,
viii. an inhibitor of Ras oncogenic isoforms,
ix. a telomerase inhibitor,
x. a protease inhibitor, a matrix metalloproteinase inhibitor, a methionine aminopeptidase inhibitor, or a proteosome inhibitor, and/or
xi) a mTOR inhibitor.

6. A S1P receptor agonist for use according to claim 4 or claim 5 for preventing or treating tumors of blood or lymphatic system or myeloid cancer.

7. A S1P receptor agonist for use according to claim 4, in combination with a chemotherapeutic agent for treating a lymphatic or myeloid cancer, wherein the chemotherapeutic agent is selected from busulfan, cytarabine, 6-thioguanine, fludarabine, hydroxyurea, procarbazine, bleomycin and methotrexate.

8. A S1P receptor agonist for use according to claim 4, in combination with a chemotherapeutic agent for treating a lymphatic cancer, wherein the chemotherapeutic agent is selected from daunorubicin and imatinib.

9. A S1 P receptor agonist for use according to any one of the preceding claims, wherein the S1P receptor agonist is administered intermittently.

10. A S1P receptor agonist as defined in claim 1 for use treating or preventing diseases mediated by the inhibition of the neo-angiogenesis process, where said diseases are selected from the group consisting of cancer, myocardial angiogenesis, wound healing and diabetic vascular dysfunction or vasculopathy.

11. A S1P receptor agonist for use according to any one of the preceding claims, wherein the S1P receptor agonist is 2-amino-2-[4-(benzyloxyphenylthio)-2- chlorophenyl]propyl-1,3-propane-diol or 2-amino-2-[4-(3-benzyloxyphenoxy)-2-chlorophenyl]propyl-1,3-propane-diol, in free from or in a pharmaceutically acceptable salt form.

## Patentansprüche

1. S1P-Rezeptoragonist, der eine Verbindung der Formel IX ist 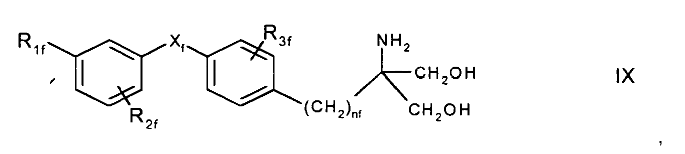 wobei
R_{1f} für Halogen, Trihalogenmethyl, Hydroxy, Niederalkyl mit 1 bis 7 Kohlenstoffatomen, Phenyl, Aralkyl, Niederalkoxy mit 1 bis 4 Kohlenstoffatomen, Trifluormethyloxy, Phenoxy, Cyclohexylmethyloxy, substituiertes oder unsubstituiertes Aralkyloxy, Pyridylmethyloxy, Cinnamyloxy, Naphtylmethyloxy, Phenoxymethyl, Hydroxymethyl, Hydroxyethyl, Niederalkylthio mit 1 bis 4 Kohlenstoffatomen, Niederalkylsulfinyl mit 1 bis 4 Kohlenstoffatomen, Niederalkylsulfonyl mit 1 bis 4 Kohlenstoffatomen, Benzylthio, Acetyl, Nitro oder Cyano steht;
R_{2f} für Wasserstoff, Halogen, Trihalogenmethyl, Niederalkoxy mit 1 bis 4 Kohlenstoffatomen, Niederalkyl mit 1 bis 7 Kohlenstoffatomen, Phenethyl oder Benzyloxy steht;
R_{3f} für Wasserstoff, Halogen, Trifluormethyl, Niederalkoxy mit 1 bis 4 Kohlenstoffatomen, Hydroxy, Benzyloxy, Niederalkyl mit 1 bis 7 Kohlenstoffatomen, Phenyl oder Niederalkoxymethyl mit 1 bis 4 Kohlenstoffatomen steht;
X_{f} für O oder S steht und
n_{f} für eine ganze Zahl von 1 bis 4 steht,
oder ein pharmazeutisch akzeptables Salz hiervon,
zur Verwendung bei einem Verfahren zum Verhüten oder Behandeln von Erkrankungen, die durch einen Neoangiogeneseprozess vermittelt werden oder mit deregulierter Angiogenese in Verbindung stehen, wobei die Erkrankungen aus der Gruppe ausgewählt sind, die aus Krebs, myokardialer Angiogenese, Wundheilung und diabetischer vaskulärer Dysfunktion oder Vaskulopathie besteht.

2. S1P-Rezeptoragonist zur Verwendung gemäß Anspruch 1 zum Verhüten oder Behandeln von festen Tumoren.

3. S1P-Rezeptoragonist zur Verwendung gemäß Anspruch 1 zum Hemmen des Wachstums von festen Tumoren oder zum Behandeln der Invasivität fester Tumoren oder von Symptomen, die mit einem derartigen Tumorwachstum verbunden sind.

4. S1P-Rezeptoragonist der Formel IX gemäß Definition in Anspruch 1, oder ein pharmazeutisch akzeptables Salz hiervon zur Verwendung bei einem Verfahren zum Verhüten oder Behandeln von Erkrankungen, die durch einen Neoangiogeneseprozess vermittelt werden oder mit einer deregulierten Angiogenese in Verbindung stehen, wobei die Erkrankungen aus der Gruppe ausgewählt sind, die aus Krebs, myokardialer Angiogenese, Wundheilung, diabetischer vaskulärer Dysfunktion oder Vaskulopathie, lymphoproliferativen und myeloproliferativen Störungen besteht, wobei der S1P-Rezeptoragonist begleitend oder in Sequenz mit einem chemotherapeutischen Mittel verabreicht wird.

5. S1P-Rezeptoragonist zur Verwendung gemäß Anspruch 4, wobei das chemotherapeutische Mittel aus
i) einem Aromatase-Inhibitor,
ii) einem Antiöstrogen, einem Antiandrogen oder einem Gonadorelin-Agonisten,
iii) einem Topoisomerase-I-Inhibitor oder einem Topoisomerase-II-Inhibitor,
iv) einem Mikrotubulus-aktiven Mittel, einem Alkylierungsmittel, einem antineoplastischen Antimetaboliten oder einer Platinverbindung,
v) einer Verbindung, die auf eine Protein- oder Lipidkinaseaktivität oder eine Protein- oder Lipidphosphataseaktivität abzielt bzw. diese verringert, einer weiteren anti-angiogenen Verbindung oder einer Verbindung, die Zelldifferenzierungsprozesse auslöst,
vi) einem Bradykinin-1-Rezeptor oder einem Angiotensin-II-Antagonisten,
vii) einem Cyclooxygenase-Inhibitor, einem Bisphosphonat, einem Histon-Deacetylase-Inhibitor, einem Heparanase-Inhibitor, einem Modifikator einer biologischen Reaktion, einem Ubiquitinierungs-Inhibitor oder einem Inhibitor, der anti-apoptotische Wege blockiert,
viii) einem Inhibitor von onkogenen Ras-Isoformen,
ix) einem Telomerase-Inhibitor,
x) einem Protease-Inhibitor, einem Matrix-Metalloproteinase-Inhibitor, einem Methionin-Aminopeptidase-Inhibitor oder einem Proteosom-Inhibitor und/oder
xi) einem mTOR-Inhibitor
ausgewählt ist.

6. S1P-Rezeptoragonist zur Verwendung gemäß Anspruch 4 oder Anspruch 5 zum Verhüten oder Behandeln von Tumoren des Bluts oder des lymphatischen Systems oder von myeloischem Krebs.

7. S1P-Rezeptoragonist zur Verwendung gemäß Anspruch 4 in Kombination mit einem chemotherapeutischen Mittel zum Behandeln eines lymphatischen oder myeloischen Krebses, wobei das chemotherapeutische Mittel aus Busulfan, Cytarabin, 6-Thioguanin, Fludarabin, Hydroxyharnstoff, Procarbazin, Bleomycin und Methotrexat ausgewählt ist.

8. S1P-Rezeptoragonist zur Verwendung gemäß Anspruch 4 in Kombination mit einem chemotherapeutischen Mittel zum Behandeln eines Krebses des lymphatischen Systems, wobei das chemotherapeutische Mittel aus Daunorubicin und Imatinib ausgewählt ist.

9. S1P-Rezeptoragonist zur Verwendung gemäß einem der vorhergehenden Ansprüche, wobei der S1P-Rezeptoragonist intermittierend verabreicht wird.

10. S1P-Rezeptoragonist gemäß Definition in Anspruch 1 zur Verwendung bei einem Verfahren zum Behandeln oder Verhüten von Erkrankungen, die durch die Hemmung des Neoangiogeneseprozesses vermittelt werden, wobei die Erkrankungen aus der Gruppe ausgewählt sind, die aus Krebs, myokardialer Angiogenese, Wundheilung und diabetischer vaskulärer Dysfunktion oder Vaskulopathie besteht.

11. S1P-Rezeptoragonist zur Verwendung gemäß einem der vorhergehenden Ansprüche, wobei der S1P-Rezeptoragonist 2-Amino-2-[4-(benzyloxyphenylthio)-2-chlorphenyl]propyl-1,3-propan-diol oder 2-Amino-2-[4-(3-benzyloxyphenoxy)-2-chlorphenyl]propyl-1,3-propan-diol in freier Form oder in Form eines pharmazeutisch akzeptablen Salzes ist.

## Revendications

1. Agoniste du récepteur de la S1P qui est un composé de formule IX 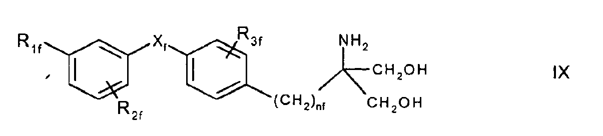 dans laquelle
R_{1f} est un halogène, un groupe trihalométhyle, hydroxy, alkyle inférieur comportant 1 à 7 atomes de carbone, phényle, aralkyle, alcoxy inférieur comportant 1 à 4 atomes de carbone, trifluorométhyloxy, phénoxy, cyclohexylméthyloxy, aralkyloxy substitué ou non substitué, pyridylméthyloxy, cinnamyloxy, naphtylméthyloxy, phénoxyméthyle, hydroxyméthyle, hydroxyéthyle, alkyle inférieur-thio comportant 1 à 4 atomes de carbone, alkyle inférieur-sulfinyle comportant 1 à 4 atomes de carbone, alkyle inférieur-sulfonyle comportant 1 à 4 atomes de carbone, benzylthio, acétyle, nitro ou cyano ;
R_{2f} est l'hydrogène, un halogène, un groupe trihalométhyle, alcoxy inférieur comportant 1 à 4 atomes de carbone, alkyle inférieur comportant 1 à 7 atomes de carbone, phénéthyle ou benzyloxy ;
R_{3f} est l'hydrogène, un halogène, un groupe trifluorométhyle, alcoxy inférieur comportant 1 à 4 atomes de carbone, hydroxy, benzyloxy, alkyle inférieur comportant 1 à 7 atomes de carbone, phényle ou alcoxy inférieur-méthyle comportant 1 à 4 atomes de carbone ;
X_{f} est O ou S ; et
n_{f} est un entier compris entre 1 et 4,
ou un sel de celui-ci, acceptable sur le plan pharmaceutique,
pour une utilisation dans un procédé de prévention ou de traitement de maladies médiées par un processus de néo-angiogenèse ou associées à un dérèglement de l'angiogenèse, lesdites maladies étant sélectionnées dans le groupe composé d'un cancer, d'une angiogenèse myocardique, d'une cicatrisation ou d'un dysfonctionnement vasculaire observé dans le diabète ou d'une vasculopathie diabétique.

2. Agoniste du récepteur de la S1P à utiliser selon la revendication 1 pour prévenir ou traiter des tumeurs solides.

3. Agoniste du récepteur de la S1P à utiliser selon la revendication 1, pour inhiber la croissance de tumeurs solides ou pour traiter le caractère invasif d'une tumeur solide ou les symptômes associés à une telle croissance tumorale.

4. Agoniste du récepteur de la S1P de formule IX selon la revendication 1, ou sel de celui-ci acceptable sur le plan pharmaceutique, pour une utilisation dans un procédé de prévention ou de traitement de maladies médiées par un processus de néo-angiogenèse ou associées à un dérèglement de l'angiogenèse, lesdites maladies étant sélectionnées dans le groupe composé d'un cancer, d'une angiogenèse myocardique, d'une cicatrisation, d'un dysfonctionnement vasculaire observé dans le diabète ou d'une vasculopathie diabétique, de troubles lymphoprolifératifs et de troubles myéloprolifératifs, où l'agoniste du récepteur de la S1P est administré de façon concomitante ou séquentielle avec un agent chimiothérapeutique.

5. Agoniste du récepteur de la S1P à utiliser selon la revendication 4, dans lequel l'agent chimiothérapeutique est sélectionné parmi
i. un inhibiteur d'aromatase,
ii. un anti-oestrogène, un anti-androgène ou un agoniste de la gonadoréline,
iii. un inhibiteur de la topo-isomérase I ou un inhibiteur de la topo-isomérase II,
iv. un agent actif sur les microtubules, un agent d'alkylation, un antimétabolite antinéoplasique ou un composé platine,
v. un composé qui cible/réduit l'activité d'une protéine ou d'une lipide kinase ou l'activité d'une protéine ou d'une lipide phosphatase, un autre composé antiangiogène ou un composé qui induit des processus de différenciation cellulaire,
vi. un récepteur de la bradykinine 1 ou un antagoniste de l'angiotensine II,
vii. un inhibiteur de la cyclooxygénase, un bisphosphonate, un inhibiteur d'histone désacétylase, un inhibiteur d'héparanase, un modificateur de la réponse biologique, un inhibiteur d'ubiquitination, ou un inhibiteur qui bloque les voies anti-apoptotiques,
viii. un inhibiteur d'isoformes oncogènes de Ras,
ix. un inhibiteur de télomérase,
x. un inhibiteur de protéase, un inhibiteur de métalloprotéinase de matrice, un inhibiteur de méthionine aminopeptidase ou un inhibiteur de protéosome, et/ou
xi) un inhibiteur de mTOR.

6. Agoniste du récepteur de la S1P à utiliser selon la revendication 4 ou la revendication 5, pour prévenir ou traiter des tumeurs du système sanguin ou lymphatique ou un cancer myéloïde.

7. Agoniste du récepteur de la S1P à utiliser selon la revendication 4, en combinaison avec un agent chimiothérapeutique pour traiter un cancer lymphatique ou myéloïde, où l'agent chimiothérapeutique est sélectionné parmi le busulfan, la cytarabine, la 6-thioguanine, la fludarabine, l'hydroxyurée, la procarbazine, la bléomycine et le méthotrexate.

8. Agoniste du récepteur de la S1P à utiliser selon la revendication 4, en combinaison avec un agent chimiothérapeutique pour traiter un cancer lymphatique, où l'agent chimiothérapeutique est sélectionné parmi la daunorubicine et l'imatinib.

9. Agoniste du récepteur de la S1P à utiliser selon l'une quelconque des revendications précédentes, dans lequel l'agoniste du récepteur de la S1P est administré de façon intermittente.

10. Agoniste du récepteur de la S1P selon la revendication 1, pour une utilisation dans un procédé de traitement ou de prévention de maladies médiées par l'inhibition du processus de néo-angiogenèse, où lesdites maladies sont sélectionnées dans le groupe composé d'un cancer, d'une angiogenèse myocardique, d'une cicatrisation et d'un dysfonctionnement vasculaire observé dans le diabète ou d'une vasculopathie diabétique.

11. Agoniste du récepteur de la S1P à utiliser selon l'une quelconque des revendications précédentes, dans lequel l'agoniste du récepteur de la S1P est le 2-amino-2-[4-(benzyloxyphénylthio)-2-chlorophényl]propyl-1,3-propane-diol ou le 2-amino-2-[4-(3-benzyloxyphénoxy)-2-chlorophényl]propyl-1,3-propane-diol, sous une forme libre ou sous la forme d'un sel acceptable sur le plan pharmaceutique.
